Europäisches Patentamt

European Patent Office

Office européen des brevets

(11) Publication number: 0 464 298 B1

# EUROPEAN PATENT SPECIFICATION

(45) Date of publication of patent specification: **21.06.95**    (51) Int. Cl.⁶: **A61K 35/78**

(21) Application number: **90830308.4**

(22) Date of filing: **05.07.90**

The file contains technical information submitted after the application was filed and not included in this specification

(54) **Echinacea extracts, a process for the preparation thereof and formulations containing them.**

(43) Date of publication of application:
**08.01.92 Bulletin 92/02**

(45) Publication of the grant of the patent:
**21.06.95 Bulletin 95/25**

(84) Designated Contracting States:
**AT BE CH DE DK ES FR GB GR IT LI LU NL SE**

(56) References cited:
**EP-A- 0 144 368**
**DE-A- 2 721 014**

**ARZNEIMITTEL-FORSCHUNG/DRUG RES., vol. 38 (1), no. 2, 1988, pages 276-281; R.BAUER et al.: "Immunologische in-vivo- und in-vitro-Untersuchungen mitEchinacea-Extrakten"**

(73) Proprietor: **INDENA S.p.A.**
**Via Ripamonti, 99**
**I-20141 Milano (IT)**

(72) Inventor: **Bombardelli, Ezio**
**Via Ripamonti, 99**
**I-20141 Milano (IT)**
Inventor: **Frangi, Enrico**
**Via Ripamonti, 99**
**I-20141 Milano (IT)**

(74) Representative: **Bianchetti, Giuseppe**
**Studio Consulenza Brevettuale,**
**Via Rossini, 8**
**I-20122 Milan (IT)**

**Description**

The present invention relates to a process for the preparation of lipophilic extracts starting from roots or aereal parts of Echinacea purpurea Moench and Echinacea angustifolia, by means of carbon dioxide under hypercritical conditions. The invention also relates to the therapeutical use of said extracts for the treatment of diseases related to prostatic benign hyperplasia, in which they act on the symptomatology as well as on inflammatory processes often deriving from retention of urine or common bacterial infections.

The two main Echinacea species are known to contain a number of strongly polyunsaturated compounds (isobutylamides) to which immuno-modulating properties have been attributed, playing a favourable role in the defence against inflammations caused by pathogens. The lipophilic extracts from Echinacea prepared according to the invention have a strong antiinflammatory activity by both the oral and the systemic routes, so as to make them extremely valuable in prostatic decongestion.

Both the isobutylamido and the polyine components present in the extract are particularly unstable substances since they easily undergo oxidation, as described in literature; in fact the extracts obtained from vegetable material which has neither previously been dried nor stored under appropriate conditions show changes and isomeric by-products in comparison with the extracts obtained from the fresh plant.

The processes described in literature for the preparation of lipophilic extracts are based on the use of aliphatic alcohols or chlorinated solvents, such as chloroform, methylene chloride, and the like; all of these solvents suffer from the drawback of extracting also a lot of hydrophilic material and, above all, on industrial scale, of lack of stability in the absence of antioxidants.

Therefore, the known Echinacea extracts hithereto obtained suffer from the presence of contaminants consisting of traces of the extraction solvent and easily undergo oxidation reactions, therefore requiring the addition of antioxidants or stabilizers to obtain reliable products which can be used in the pharmaceutical and/or cosmetical fields, to the detriment of the therapeutic activity of the extracts.

On the contrary, the lipophilic extracts from Echinacea prepared according to the invention have a strong antiinflammatory activity by both the topical and the systemic routes, so as to be very useful in prostatic decongestion.

In fact, compared with the processes of the prior art, the process of the present invention, involving the use of carbon dioxide, allows to obtain the following essential advantages:

. selective extraction, under quite mild conditions, of the active ingredients which turn out to be uncontaminated by undesired side-products, above all in case of extraction of the aereal parts of the plant, which contain remarkable amounts of chlorophyl and derivatives thereof, besides the active principles;

. prevention of alterations of the extract (oxidations) due to the presence of $CO_2$ which acts as a deareation agent and therefore as a preservative;

. achievement of extracts which are safe and free from residues involving laborious purifications.

The process of the present invention involves the use of pure carbon dioxide, at temperatures of 35-55°C and under pressures ranging from 110 to 220 bars,; the evaporation of the solvent in the concentrator is carried out varying the operative conditions, lowering temperature to about 25°C and pressure to 50 bars. The lipophilic extract is concentrated in the condenser at room temperature, in form of a pale yellow-beige semi-fluid residue, having an isobutylamide content varying from 25 to 75%, depending on the plant part and species which have been used.

The control of the isobutylamide content is carried out either by gas chromatography, according to procedures hereinbelow described, or by high pressure liquid chromatography, according to the procedures reported in literature (Deutsche Apotheker Zeitung, 4, 174-80, 1988). For chromatographic determination, fused silica columns are used (15 m x 0.32 mm i.d.) coated with SE-30 (layer thickness 0.072 μm) using helium as the carrier gas at 1.5 ml/min.; the column temperature is increased from 100°C to 350°C, the detector temperature is 350°C. The sample, before the injection, is silanized using a 1:1:1 mixture of Sylon BTZ, BSTFA and pyridine as the reagent; as the calibration standard, synthetic pellitorine is used which, independently on the unsaturation degree, is chemically comparable but more stable.

The products of the invention show, from the pharmacological point of view, a remarkable antiinflammatory activity both by the epicutaneous and the systemic administrations. The results of antiedematous activity tests in the mouse, after epicutaneous administration of the extract according to the procedures reported in Agents and Actions (17, 347-49, 1985) are shown in the following table :

## Table I:

Antiedemic activity of the lipophilic extract from Echinacea angustifolia in the croton oil test in the mouse.

| Substance | Dose µg | edema mg* | reduction percent | p |
|---|---|---|---|---|
| Controls | — (26) | 7.9+ 0.3 | — | — |
| $CO_2$ extract | 180 (13) | 4.4+ 0.4 | 45.7 | 0.001 |
| | 90 (28) | 5.5+ 0.3 | 31.0 | 0.001 |
| | 9 (14) | 8.1+ 0.3 | −1.0 | n.s. |
| Isobutylamide** | 80 (28) | 4.1+ 0.2 | 49.3 | 0.001 |
| | 40 (28) | 4.4+ 0.3 | 31.6 | 0.001 |
| | 20 (28) | 5.8+ 0.3 | 26.2 | 0.001 |
| Hydrocortisone | 30 (14) | 4.5+ 0.2 | 44.1 | 0.001 |

The number of used animals is in brackets.

* The edema values are expressed as mean ± standard error

p at the variance analysis

**Dodeca-(2E,4E,8Z,10E)tetra-en-carboxy-(1)-isobutylamide.

The determinations have been carried out 6 hours after the administration of the inflammatory agent.

## Table II.

Antiinflammatory test of the lipophilic extract from Echinacea purpurea in the carrageenin edema test in the rat.

| Substance | Dose mg/kg/os | Edema 0h | Edema 1h | Edema 2h | Edema 4h |
|---|---|---|---|---|---|
| Controls | --(10) | 1.71 | 1.87 | 2.25 | 2.47 |
| $CO_2$ extract* | 20 (10) | 1.68 | 1.82 | 1.91 | 1.98 |
| | | | -12% | -57% | -60% |

The products have been administered one hour before carrageenin in the paw of Wistar male rats of 200 g body weight.

* extract obtained by extraction from Echinacea purpurea roots.

The immuno-modulating activity of the extracts prepared according to the invention has been tested in the macrophages stimulation test, in the test of antibody hemoagglutination induced by sheep erythrocytes in the mouse and in leukocytes and killer splenocytes in the same animal. Table III shows the data of the splenocyte activation in the mouse.

**<u>Table III:</u>**

Killer activity of splenocytes of mouse treated with a lipophilic extract from Echinacea purpurea.

---------------------------------------------------------------

| Test n°. | mice treatment | % specific lysis |
|---|---|---|
| I | a) physiological saline | 52 |
| | b) lipophilic extr. from Echinacea purpurea | 92** |
| II | a) physiological saline | 52 |
| | b) lipophilic extr. from Echinacea purpurea | 85** |

---------------------------------------------------------------

** $P<0.01$ t test for paired data.

Splenocytes from 5 animals are pooled and tested. In the prostatic field, the activity control has been carried out on rats in which an experimental hyperplasia had been induced, hystologically checking the effects of the extracts on prostate function; a flattening of the glandular epithelia and a dilatation of the glandular tubuli, which are signs of an improved function of the organ, have been evidenced in experimentally hyperplasic animals which had simultaneously been administered with the lipophilic extracts from Echinacea angustifolia or purpurea.

Patients treated with doses from 20 to 200 mg/day of extract and showing a clinical picture with dysuria, pollakiuria and postmictional residual urine and other typical complications of hyperplasia got a remarkable benefit from the administration of the drug for a period from 8 to 24 days.

The present invention also relates to pharmaceutical compositions containing as the active principle the extracts obtained according to the process of the invention itself.

Said extracts can be vehicled in the traditional pharmaceutical formulations, such as those reported in Remington' Pharmaceutical Sciences Handbook, Hack Pub. Co., N.Y.,USA; amongst the not conventional excipients, cyclodextrins proved to be particularly useful, since they form with unsaturated isobutylamides hydrophilic stable complexes which allow a controlled release. Oily oral formulations in soft gelatin capsules and suppository formulations turned out to be particularly interesting.

The following not limiting examples further illustrate the present invention.

EXAMPLE 1

Preparation of the lipophilic extract from Echinacea angustifolia.

1.35 kg of finely ground roots of Echinacea angustifolia are extracted in a 5 liter extractor fitted with a heating and cooling mantle and with the equipment for the control of high pressures. The vegetable material is extracted using 20 l of continuously recycled liquid carbon dioxide for 3 hours at a temperature of 40°C and under a pressure of 200 bars, the conditions in the evaporator being 25°C and 50 bars.

After evaporation of the solvent, 15.5 g of a pasty yellow-beige product were obtained, having an isobutylamide content of about 50%.

EXAMPLE 2

Preparation of a lipophilic extract from Echinacea purpurea leaves and flowers.

0.9 kg of finely ground aereal parts of Echinacea purpurea are placed into a 5 liter extractor fitted with a heating and cooling mantle in a gas permeable vessel. The vegetable material is continuously extracted for 6 hours at a temperature of 35°C and under a pressure of 140 bars and respectively 25°C and 50 bars in the evaporator.

After evaporation of the solvent, 27 g of a semi-fluid yellow mass are obtained which contains water; the product is dried overnight under vacuum at 40°C. 18.5 g of a waxy compound are obtained, having an isobutylamide content of 40%.

EXAMPLE 3

Preparation of a complex of the isobutylamides extracted from Echinacea purpurea with beta-cyclodextrin.

15 g of beta-cyclodextrin are dissolved in 1.5 liter of demineralised water at room temperature; 4 g of the lipophilic extract prepared according to Example 2 dissolved in 10 ml of ethanol are added to the solution. The resulting oily suspension is sonicated for 5 hours, until the suspended oily material disappears; during sonication, an abundant whitish precipitate forms which is filtered after standing overnight in refrigerator. This residue, after drying under vacuum for a night at 50°C, weighs 12.6 g and consists of a beta-cyclodextrin - isobutylamide complex and similar products. The formation of these complexes can be proved by means of solubility and NMR determinations.

EXAMPLE 4

Equimolecular complex of pellitorine with alpha-cyclodextrin.

2.23 g of pellitorine are added under strong stirring to a solution of 9.7 g of alpha-cyclodextrin in 200 ml of water, the suspension is sonicated for 8 hours, then lyophilized.

11.9 g of a white solid are obtained, having spectroscopical characteristics corresponding to those of a complex.

**Claims**

**Claims for the following Contracting States : DE, GB, FR, IT, NL, SE, LI, CH, BE, AT, LU, DK**

1. A process for the preparation of Echinacea lipophilic extracts, in which process $CO_2$ under hypercritical conditions is used as the extraction solvent, which is subsequently evaporated off.

2. A process as claimed in claim 1, in which process roots or aereal parts of Echinacea purpurea Moench or of Echinacea angustifolia are subjected to extraction.

3. A process as claimed in claims 1 and 2, in which process the extraction is carried out at temperatures from 35 to 55°C and under pressures from 110 to 220 bars, and the evaporation is carried out at temperatures of about 25°C and under pressures of about 50 bars.

4. A process as claimed in the preceding claims, in which process the obtained extract can subsequently be subjected to dehydration.

5. A process as claimed in any one of the preceding claims, which process is carried out continuously.

6. Echinacea lipophilic extracts, which can be obtained by means of the extraction processes as claimed in claims 1 to 5.

7. Pharmaceutical compositions having immuno-modulating, antiinflammatory and antiedemic activities, containing as the active principles the Echinacea lipophilic extracts as claimed in claim 6, optionally in admixture with conventional carriers or excipients.

6

8. Pharmaceutical compositions as claimed in claim 7, for the oral, parenteral or rectal administrations.

9. Pharmaceutical compositions as claimed in claim 8, for the prolonged oral administration containing a complex of isobutylamides extracted from Echinacea purpurea with beta-cyclodextrin.

**Claims for the following Contracting States : ES, GR**

1. A process for the preparation of Echinacea lipophilic extracts, in which process $CO_2$ under hypercritical conditions is used as the extraction solvent, which is subsequently evaporated off.

2. A process as claimed in claim 1, in which process roots or aereal parts of Echinacea purpurea Moench or of Echinacea angustifolia are subjected to extraction.

3. A process as claimed in claims 1 and 2, in which process the extraction is carried out at temperatures from 35 to 55°C and under pressures from 110 to 220 bars, and the evaporation is carried out at temperatures of about 25°C and under pressures of about 50 bars.

4. A process as claimed in the preceding claims, in which process the obtained extract can subsequently be subjected to dehydration.

5. A process as claimed in any one of the preceding claims, which process is carried out continuously.

**Patentansprüche**
**Patentansprüche für folgende Vertragsstaaten : DE, GB, FR, IT, NL, SE, LI, CH, BE, AT, LU, DK**

1. Verfahren zur Herstellung von lipophilen Echinaceaextrakten, wobei $CO_2$ unter hyperkritischen Bedingungen als Extraktionslösungsmittel verwendet wird, das anschließend abgedampft wird.

2. Verfahren nach Anspruch 1, wobei Wurzeln oder an der Luft befindliche Teile von Echinacea purpurea Moench oder von Echinacea angustifolia extrahiert werden.

3. Verfahren nach Anspruch 1 und 2, wobei das Extraktionsverfahren bei einer Temperatur von 35 bis 55°C und unter Drücken von 110 bis 220 bar durchgeführt wird und die Verdampfung bei einer Temperatur von etwa 25°C und unter Drücken von etwa 50 bar durchgeführt wird.

4. Verfahren nach einem der vorausgehenden Ansprüche, wobei der erhaltene Extrakt anschließend entwässert werden kann.

5. Verfahren nach einem der vorausgehenden Ansprüche, wobei das Verfahren kontinuierlich durchgeführt wird.

6. Lipophile Echinaceaextrakte, die mittels der Extraktionsverfahren nach den Ansprüchen 1 bis 5 erhalten werden können.

7. Pharmazeutische Präparate mit immunmodulierenden, antiinflammatorischen und antiödematösen Eigenschaften, enthaltend als Wirkstoffe die lipophilen Echinaceaextrakte nach Anspruch 6, gegebenenfalls im Gebrauch mit herkömmlichen Trägern oder Excipientien.

8. Pharmazeutische Präparate nach Anspruch 7 für die oralen, parenteralen oder rektalen Verabreichungen.

9. Pharmazeutische Präparate nach Anspruch 8 für die prolongierte orale Verabreichung, umfassend einen aus Echinacea purpurea mit Beta-Cyclodextrin extrahierten Komplex aus Isobutylamiden.

**Patentansprüche für folgende Vertragsstaaten : ES, GR**

1. Verfahren zur Herstellung von lipophilen Echinaceaextrakten, wobei $CO_2$ unter hyperkritischen Bedingungen als Extraktionslösungsmittel verwendet wird, das anschließend abgedampft wird.

**2.** Verfahren nach Anspruch 1, wobei Wurzeln oder an der Luft befindliche Teile von Echinacea purpurea Moench oder von Echinacea angustifolia extrahiert werden.

**3.** Verfahren nach Anspruch 1 und 2, wobei das Extraktionsverfahren bei einer Temperatur von 35 bis 55°C und unter Drücken von 110 bis 220 bar durchgeführt wird und die Verdampfung bei einer Temperatur von etwa 25°C und unter Drücken von etwa 50 bar durchgeführt wird.

**4.** Verfahren nach einem der vorausgehenden Ansprüche, wobei der erhaltene Extrakt anschließend entwässert werden kann.

**5.** Verfahren nach einem der vorausgehenden Ansprüche, wobei das Verfahren kontinuierlich durchgeführt wird.

**Revendications**

**Revendications pour les Etats contractants suivants : DE, GB, FR, IT, NL, SE, LI, CH, BE, AT, LU, DK**

**1.** Procédé pour la préparation d'extraits lipophiles d'Echinacea. dans lequel du $CO_2$ en conditions hypercritiques est utilisé comme solvant d'extraction, qui est ensuite éliminé par évaporation.

**2.** Procédé comme revendiqué dans la revendication 1, dans lequel les racines ou les parties aériennes d'Echinacea purpurea Moench ou Echinacea angustifolia sont soumises à l'extraction.

**3.** Procédé comme revendiqué dans les revendications 1 et 2, dans lequel l'extraction est effectuée à des températures comprises entre 35 et 55°C et à des pressions comprises entre 110 et 220 bars, et dans lequel l'évaporation est effectuée à des températures d'environ 25°C et à des pressions d'environ 50 bars.

**4.** Procédé comme revendiqué dans les revendications précédentes, dans lequel l'extrait obtenu peut ensuite être soumis à une déshydratation.

**5.** Procédé comme revendiqué dans l'une quelconque des revendications précédentes, ce procédé étant mis en oeuvre en continu.

**6.** Extraits lipophiles d'Echinacea, qui peuvent être obtenus au moyen des processus d'extraction tels que revendiqués dans les revendications 1 à 5.

**7.** Compositions pharmaceutiques ayant des activités immunomodulatrices, anti-inflammatoires et anti-oedémiques, contenant comme principes actifs les extraits lipophiles d'Echinacea tels que revendiqués dans la revendication 6, éventuellement en mélange avec des véhicules ou des excipients conventionnels.

**8.** Compositions pharmaceutiques telles que revendiquées dans la revendication 7, pour administrations orales, parentérales ou rectales.

**9.** Compositions pharmaceutiques telles que revendiquées dans la revendication 8, pour l'administration orale prolongée, contenant un complexe d'isobutylamides extraits d'Echinacea purpurea avec de la bêta-cyclodextrine.

**Revendications pour les Etats contractants suivants : ES, GR**

**1.** Procédé cour la préparation d'extraits lipophiles d'Echinacea, dans lequel du $CO_2$ en conditions hypercritiques est utilisé comme solvant d'extraction, qui est ensuite éliminé par évaporation.

**2.** Procédé comme revendiqué dans la revendication 1, dans lequel les racines ou les parties aériennes d'Echinacea purpurea Moench ou Echinacea angustifolia sont soumises à l'extraction.

**3.** Procédé comme revendiqué dans les revendications 1 et 2, dans lequel l'extraction est effectuée à des températures comprises entre 35 et 55°C et à des pressions comprises entre 110 et 220 bars, et dans lequel l'évaporation est effectuée à des températures d'environ 25°C et à des pressions d'environ 50 bars.

4. Procédé comme revendiqué dans les revendications précédentes, dans lequel l'extrait obtenu peut ensuite être soumis à une déshydratation.

5. Procéde comme revendiqué dans l'une quelconque des revendications précédentes, ce procédé étant mis en oeuvre en continu.